# EUROPEAN PATENT SPECIFICATION

(11) **EP 0 429 497 B1**
(45) Date of publication and mention of the grant of the patent: **06.10.1993**
(21) Application number: 89909072.4
(22) Date of filing: 02.08.1989
(51) Int. Cl.: C12N 15/40, C12N 15/82, C12N 1/21, C12N 5/14, A01H 4/00, A01N 63/00

(54) **COCUMBER MOSAIC VIRUS COAT PROTEIN GENE**
CUCUMBER-MOSAIKVIRUS-HÜLLPROTEINGEN
GENE DE LA PROTEINE DE L'ENVELOPPE DU VIRUS DE LA MOSAIQUE DU CONCOMBRE

(30) Priority: 19.08.1988 US 234404
(43) Date of publication of application: 05.06.1991
(73) Proprietor: THE UPJOHN COMPANY, Kalamazoo, Michigan 49001 (US); CORNELL RESEARCH FOUNDATION, INC., Ithaca, NY 14850 (US)
(72) Inventor: QUEMADA, Hector, D., Parchment, MI 49004 (US); SLIGHTOM, Jerry, L., Kalamazoo, MI 49007 (US); GONSALVES, Dennis, Geneva, NY 14456 (US); KEARNEY, Chris, Geneva, NY 14456 (US)
(74) Representative: Perry, Robert Edward
(86) International application number: US8903288
(87) International publication number: WO9002185

(56) References cited:
- EP-A- 0 223 452
- Journal of Cellular Biochemistry, UCLA, Symposium on Molecular & Cellular Biology, Abstracts of the 17th Annual Meeting, 28 February - 10 April 1988, Supplement 12C, 1988, Alan R. Liss, Inc. (New York, US), "Molecular biology of plant-pathogen interactions"; H. Quemada et al.: "The nucleotide sequence of cDNA clones from RNA 3 of cucumber mosaic virus strains C and WL", p. 287, abstract No. Y 333
- Biotechnology, vol. 6, no. 5, May 1988, (New York, N.Y. US), M. Cuozzo et al.: "Viral protection in transgenic tobacco plants expressing the cucumber mosaic virus coat protein or its antisense RNA", pp. 549-555

## Description

This invention relates to a coat protein gene of cucumber mosaic virus strain WL (CMV-WL). More specifically the invention relates to a process for preparing said gene as well as its incorporation into a transfer vector and to its use to produce transformed plant cells and transformed plants which are resistant to CMV viral infections.

### BACKGROUND OF THE INVENTION

Cucumber mosaic virus (CMV) is a single-stranded (+) RNA plant virus which has a functionally divided genome. The virus genome contains four RNA species designated RNAs1-4; 3389 nucleotides (nt), 3035 nt, 2193 nt and 1027 nt, respectively (Peden and Symons, 1973; Gould and Symons, 1982; Rezaian et al., 1984; Rezaian et al., 1985). Only RNAs1-3 are required for infectivity (Peden and Symons, 1973) because the coat protein, which is encoded by RNA 4, is also encoded by RNA 3. Translations of CMV RNAs yield a 95KDal polypeptide from RNA 1, a 94kDal polypeptide from RNA 2, (Gordon et al., 1983) and two polypeptides from RNA 3: its 5' end encodes a 35KDal polypeptide, and its 3' end encodes a 24.5kDal polypeptide (Gould and Symons, 1982). The 24.5kDal polypeptide is identical to that encoded by RNA 4 and is the coat protein.

Several strains of cucumber mosaic virus have been classified using serology (Devergne and Cardin, 1973, 1975), host range (Marrow et al. 1975), peptide mapping (Edwards and Gonsalves, 1983), and nucleic acid hybridization (Piazzola et al., 1979; Gonda and Symons, 1978). These CMV strains can be divided into two groups designated S and WT. The genome of the CMV-Q strain has been completely sequenced (Rezaian et al., 1984, 1985; Gould and Symons, 1982; Davies and Symons, 1988). The Q strain is a member of the S group, which consists of three members. The WT group is known to contain at least 17 members. From nucleotide sequence analysis and comparisons of the coat protein genes from CMV-C and CMV-WL (Quemada et al, manuscript in preparation; see Chart 1) we have determined that the C strain belongs to the WT group while the WL belongs to the S group. The nucleotide and amino acid sequences of the coat protein genes from strains C and WL differ by 22.7% and 16%, respectively (see charts 2 and 3).

As has been shown for several viruses [tobacco mosaic virus (Powell-Abel et al., 1986), alfalfa mosaic virus (Loesch-Fries et al., 1987; Tumer et al., 1987), cucumber mosaic virus (Cuozzo et al., 1988; Quemada and Slightom, in preparation), and potato virus X (Hemenway et al., 1988)] expression of the coat protein in transgenic plants results in a plant which is resistant to infection by the respective virus. However, whether this engineered cross-protection will extend to all strains of a particular virus has not been determined. The two CMV groups appear to differ in their coat protein gene by about 16%, thus it is possible that the expression of both virus coat proteins may be needed to ensure engineered cross-protection against CMV infections which could be expected under field conditions.

The CMV coat protein gene does not contain the signals necessary for its expression once transferred and integrated into a plant genome. It must be engineered to contain a constitutive promoter 5' to its translation initiation codon (ATG) and a poly(A) addition signal (AATAAA) 3' to its translation termination codon. Several promoters which function in plants are available, but we believe that the best promoters is the 35S constitutive promoters from cauliflower mosaic virus (CaMV). The poly (A) signal can be obtained from the CaMV 35S gene or from any number of well characterized plant genes, i.e. nopaline synthase (Bevan et al.,1983), octopine synthase (Depicker et al., 1982), and the bean storage protein gene phaseolin (Slightom, et al., 1983). The constructions are similar to that used for the expression of the CMV-C coat protein in PCT Patent Application PCT/US88/04321, published on 29 June 1989 as WO 89/05858, claiming the benefit of U.S. SN 135,591, filed 21 December 1987, entitled "Cucumber Mosaic Virus Coat Protein Gene", the essential parts which are herein incorporated by reference.

### INFORMATION DISCLOSURE

Plants that are resistant to virus diseases and methods for producing them are described in EP 223,452.

Information contained in the following references also describe materials, procedures, and results of interest for engineering virus coat protein genes for expression in transgenic plants.

An, G., et al. (1985) "New Cloning Vehicles for Transformation Of Higher Plants". EMBO J. 4:277-284.

An, G. (1986) "Development of plant promoter expression vectors and their use for analysis of differential activity of nopaline synthase promoter in transformed tobacco cells". Plant Physiol. 81:86-89.

Bevan, M., et al. (1983) "Structure and transcription of the nopaline synthase gene region of T-DNA". Nucleic Acids Research 11:369-379.

Cuozzo, M., et al. (1988) "Viral protection in transgenic tobacco plants expressing the cucumber mosaic virus coat protein or its antisense RNA". Bio/tech. 6:549-557.

Davies, C. and Symons, R. (1988) "Further implications for the evolutionary relationships between tripartite plant viruses based on cucumber mosaic virus RNA 3. Virology 164:in press.

Depicker, A., et al. "Nopaline synthase: transcript mapping and DNA sequence". J. Mol. Appl. Genet. 1:561-573.

Devergne, J. and Cardin, L. (1973) "Contribution a l'etude du virus de la mosaique du concombre (CMV). IV. Essai de classification de plusieurs isolats sur la base de leur structure antigenique". Ann. Phytopathol. 5:409-430.

Devergne, J. and Cardin, L. (1975) "Relations serologiques entre cucumovirus (CMV, TAV, PSV)" Ann. Phytopathol. 7:255-276.

Dodds, J.A., et al. (1985) "Cross protection between strains of cucumber mosaic virus: effect of host and type of inoculum on accumulation of virions and double-stranded RNA of the challenge strain". Virology 144:301-309.

Edwards, M. and Gonsalves, D. (1983) "Grouping seven biologically defined isolates of cucumber mosaic virus by peptide mapping". Phytopathology 73:1117-1120.

Gonda, T. and Symons, R. (1978) "The use of hybridization analysis with complementary DNA to determine the RNA sequence homology between strains of plant viruses: Its application to several strains of cucumoviruses". Virology 88:361-370.

Gonsalves, D., et al. (1982) "Tomato whiteleaf: The relation of an apparent satellite RNA and cucumber mosaic virus". Phytopathology 72:1533-1538.

Gordon, K., et al. (1982) "Highly purified cucumber mosaic virus-induced RNA-dependent RNA polymerase does not contain any of the full length translation products of the genomic RNAs". Virology 123:284-295.

Gould, A. and Symons, R. (1982) "Cucumber mosaic virus RNA 3. Determination of the nucleotide sequence provides the amino acid sequence of protein 3A and viral coat protein". Eur. J. Biochem. 126:217-227.

Hemenway, C., et al. (1988) "Analysis of the mechanism of protection in transgenic plants expressing the potato virus X coat protein or its antisense RNA". EMBO J. 7:1273-1280.

Hepburn, A., et al. (1985) "The use of pNJ5000as an intermediate vector for genetic manipulation of Agrobacterium Ti-plasmids". J. General Microbio. 131:2961-2969.

Loesch-Fries, S., et al. (1987) "Expression of alfalfa mosaic virus RNA 4 in transgenic plants confers virus resistance". EMBO J. 6:1845-1851.

Marrou, J., et al. (1975) "Caracterisation de douze souches du VMC par leurs aptitudes pathogenes: Tentative de classification". Meded. Fac. Landbouwwet. Rijks. Univ. Gent. 40:107-122.

Peden, K. and Symons, R. (1973) "Cucumber Mosaic Virus Contains a functionally divided genome". Virology 53:487-492.

Piazzola, P., et al. (1979) "Nucleic acid homologies of eighteen cucumber mosaic virus isolates determined by competition hybridization". J. Gen. Virol. 45:361-369.

Pietrzak, M., et al. (1986) "Expression in plants of two bacterial antibiotic resistant genes after protoplast transformation with a new plant expression vector". Nuc. Acids Res 14:5857-5868.

Polites, H. and Marotti, K. (1986) "A step-wise protocol for cDNA synthesis". Biotechniques 4:514-520.

Powell-Abel, P., et al. (1986) "Delay of disease development in transgenic plants that express the tobacco mosaic virus coat protein gene". Science 232:738-743.

Rezaian, M., et al. (1984) "Nucleotide sequence of cucumber mosaic virus RNA 2 reveals a translation product significantly homologous to corresponding proteins of other viruses". Eur. J. Biochem. 143:277-284.

Rezaian, M., et al. (1985) "Nucleotide sequence of cucumber mosaic virus RNA 1. Presence of a sequence complementary to part of the viral satellite RNA and homologies with other viral RNAs. Eur. J. Biochem. 150:331-339.

Slightom, J., et al. (1983) "Complete nucleotide sequence of a French bean storage protein gene:Phaseolin". Proc. Natl. Acad. Sci. U.S.A. 80:1897-1901.

Tumer, N., et al. (1987) "Expression of alfalfa mosaic virus coat protein gene confers cross-protection in transgenic tobacco and tomato plants". EMBO J. 6:1181-1188.

Vilaine, F. and Casse-Delbart, F. (1987) "Independent induction of transformed roots by the TL and TR region of the Ri plasmid of agropine type Agrobacterium rhizogenes". Mol. Gen. Genet. 206:17-23.

### SUMMARY OF THE INVENTION

This invention provides: The coat protein gene from the WL strain of cucumber mosaic virus (CMV-WL).

A plant transformation vector comprising the coat protein gene from CMV-WL, the promoter of the 35S gene of cauliflower mosaic virus and the polyadenylation signal of cauliflower mosaic virus 35S gene.

A bacterial cell containing a plant transformation vector comprising the coat protein gene from CMV-WL, the 35S promoter of cauliflower mosaic virus and the polyadenylation signal of the cauliflower mosaic virus 35S gene.

A transformed plant cell containing the coat protein gene from CMV-WL, the cauliflower mosaic virus 35S promoter and the polyadenylation signal of the cauliflower mosaic virus gene.

A plant comprising transformed cells containing the coat protein gene of CMV-WL, the cauliflower mosaic virus 35S promoter and the polyadenylation signal of the cauliflower mosaic virus gene. Transformed plants of this invention include beets, citrus fruit, corn, cucumber, peppers, potatoes, soybean, squash and tomatoes. Especially preferred are members of the cucurbitaceae (squash, cucumber, i.e.,) and solanaceae (peppers, tomatoes, i.e.) family.

A process for producing virus-resistant plants comprising propagating a plant expressing the coat protein gene from the WL strain of cucumber virus. Especially preferred is the process for producing members of the cucurbitacaea and solanacaea families.

### DESCRIPTION OF THE PREFERRED EMBODIMENT

Charts 1 to 5 are set forth to illustrate the constructions of this invention. Certain conventions are used to illustrate plasmids and DNA fragments as follows:
(1) The single line figures represent both circular and linear double-stranded DNA.
(2) Asterisks (*) indicate that the molecule represented is circular. Lack of an asterisk indicates the molecule is linear.
(3) Junctions between natural boundaries of functional components are indicated by vertical lines along the horizontal lines.
(4) Genes or functional components are indicated.
(5) Distances between genes and restriction sites are not to scale. The figures show the relative positions only unless indicated otherwise.

Most of the recombinant DNA methods employed in practicing the present invention are standard procedures, well known to those skilled in the art, and described in detail, for example, EP-223452 which is incorporated herein by reference. Enzymes are obtained from commercial sources and are used according to the vendor's recommendations or other variations known to the art. Reagents, buffers, and culture conditions are also known to those in the art. General references containing such standard techniques incorporated herein by reference include the following: R.Wu, ed. (1979) Methods in Enzymology Vol.68; J.H. Miller (1972) Experiments in Molecular Genetics; T. Maniatis et al.(1982) Molecular Cloning: A Laboratory Manual; and D.M. Glover, ed. (1985) DNA Cloning Vol II; S.B. Gelvin and R.A. Schilperoort, eds. Introduction, Expression, and Analysis of Gene Products in Plants.

### Example 1 Isolation of CMV RNAs

Cucumber mosaic virus strain WL (CMV-WL) was propagated in tobacco plants (cv. Havana 423) and RNA was isolated by standard methods, for example by the method of Lot et al. (Annals of Phytopathology 4:25, 1972). RNA 3 was separated from other CMV-WL RNAs by sucrose density gradient centrifugation.

### Example 2 Cloning of CMV-WL RNA3

### (a) Synthesis of double-stranded cDNA3

Purified CMV-WL RNA3 was polyadenylated in order to provide a site for the annealing of an oligo dT primer. The reaction buffer was as follows: 5 µl, 1 M Tris pH 7.9; 1 µl, 1 M MgCl₂; 2.5 µl, 0.1 M MnCL₂; 5 µl, 5 µ NaCl; 0.5 µl, 100 mM ATP; 18 µl, 2.8 mg/ml bovine serum albumin. 3.2 µl of this buffer were mixed with 2 µg of CMV-WL RNA3. 3.8µl H₂O and 1 µl of poly-A polymerase were added, and the reaction mixtures were incubated at 37°C. for 10 minutes.

The resulting polyadenylated RNA was used in the cDNA synthesis protocol of Polites and Marotti (Biotechniques 4:514, 1986), except that 0.75 mM KCl was used instead of 50 mM NaCl, and 130 uCi/100 µl ³²p-dCTP was used instead of 10-50 uCi/100 µl·

After ds-cDNA was synthesized, it was purified by G-100 column chromatography, precipitated with ethanol, and suspended in 20 µl of 1X Eco R1 methylase buffer (100 nM NaCl, 100 mM Tris-HCL pH 8.0, 1 mM EDTA,80 µM S-adenosyl methionine, 100 µg/ml bovine serum albumin). After removal of a 2 µl aliquot for subsequent gel analysis, an additional 1 µl of 32 mM S-adenosyl methionine was added to the reaction mixture mix, and 1 µl (20 units) of Eco RI methylase. The reaction was incubated at 37°C. for 30 minutes and stopped by incubation at 70°C. for 10 minutes.

Two µl were removed from the above reaction, and 1 µl (5 units) of E. coli DNA polymerase I klenow fragment was added. The reaction was incubated at 37°C. for 10 minutes, then extracted with phenol/chloroform before precipitating with ethanol. The pellet was washed in 70% ethanol, then in 70% ethanol/0.3 M sodium acetate.

The pellet was dried and resuspended in 8 µl 0.5 µg/µl phosphorylated Eco RI linkers (available from Collaborative Research, Inc, 128 Spring Street, Lexington, MA 02173). One µl 10X ligase buffer (800 mM Tris-HCl pH 8.0, 200 mM MgCl₂, 150 mM DTT, 10 mM ATP) and 1 µl of T4 DNA ligase (4 units/µl) were added, and the reaction was incubated overnight at 15°C.

The ligation reaction was then stopped by incubation at 65°C. for 10 minutes. Sixty µl of water, 10 µl of 10X Eco RI salts(900 mM Tris pH 8.0, 100 mM MgCl2, 100 mM NaCl), and 10 µl of EcoRI (10 units/µl) were added, and the reaction was incubated at 37°C. for 1 hr (a 5 µl aliquot was removed at the beginning for subsequent gel analysis). The reaction was stopped by phenol/chloroform and chloroform extraction. A 5 µl aliquot was removed for gel analysis, and half of the remainder was frozen for future use. The other half was purified by G-100 column chromatography. The G-100 fractions containing the cDNA were concentrated by butanol extraction, precipitated with ethanol, and resuspended in 10 µl of H₂O. After removing 3 µl for subsequent analysis, 1 µl lambda gtll or lambda Zap arms (available from Stratagene Co., 3770 Tandy St, San Diego, Ca. 92121), 1 µl of 10X ligase buffer, and 1 µl T4 DNA ligase were added, and the reaction was incubated at 15°C. overnight.

The resulting ligated lambda gtll or Zap/cDNA molecules were packaged according to the procedure recommended by the manufacturer of the packaging extract (Gigapack plus, also from Stratagene). This yielded recombinant lambda phage, which were plated according to methods known to those skilled in the art.

Lambda clones containing the coat protein gene were identified by hybridization with radioactively labelled single-stranded cDNA from purified RNA4 of CMV-WL. This RNA4 single-stranded cDNA was synthesized as follows: RNA 4 molecules were polyadenylated as described above for CMV-WL RNA3, except that 5.8 µg RNA4 was used. First strand synthesis was as described by Polites and Marotti (Biotechniques 4:514, 1986) except that non-radioactive dCTP was not included. Instead, 260 uCi/100 µl of radioactive dCTP was used.

The labelled single-stranded cDNA was purified by P6 column chromatography and used to probe replicate filters lifted from the lambda phage plates mentioned above. The single-stranded cDNA hybridized with DNA from several phage clones, indicating that they contained at least a part of the CMV-WL coat protein gene. Several of these lambda clones were grown, and DNA from them was isolated according to methods known to those skilled in the art. In particular, lambda clone WL3Z8 was isolated and its insert of about 2.0kb contains all of CMV-WL RNA3 except the 5'-193 bp.

### Example 3 Construction of a pUC19 Clone containing the CMV-WL coat protein gene

The EcoRI fragments from lambda clone WL3Z8 were transferred to the plasmid vector, pUC19 (available from Bethesda Research, P.O. Box 6009, Gaithersburg, Md 20877), using standard methods to obtain clone pWL3Z8.1. The EcoRI cloned fragments in pUC19 were then sequenced by the technique described by Maxam and Gilbert (Methods in Enzymology 65:499, 1980). Based on this information the complete sequence of the CMV-WL coat protein gene was determined and this is shown in Chart 1. Additional sequencing showed that clone pWL3Z8.1 contains all but the 5'193 bp of the CMV-WL RNA3 molecule, as determined by comparison with the complete sequence of CMV-Q RNA3 (Davis and Symons, 1988, Virology 164:in press). The nucleotide and amino acid sequence of CMV-WL and CMV-C differ by 22.7 % (chart 2) and 16% (chart 3), respectively.

### Example 4 Construction of a micro T-DNA plasmid containing a plant-expressible CMV-WL coat protein gene with the CaMV 35S polyadenylation signal

In order to attach the CaMV 35S promoter and polyadenylation signal, a fragment extending from an ApaI site (located within the intergenic region of RNA3) to an EcoRI site (attached during the cloning experiment) was removed from lambda clone WL3Z8 and ligated into the multiple cloning site of the vector pDH51 (Pietrzak et al., 1986) (available from Thomas Hohn, Friedrich Miescher Institute, P.O. Box 2543, CH-4002, Basel, Switzerland). This was accomplished by complete digestion with ApaI and a partial digest with EcoRI of WL3Z8, creating a blunt-ended molecule out of the appropriate ApaI to EcoRI 1090 bp fragment (using mung bean nuclease), followed by ligating it into the SmaI site of pDH51 (see Chart 4). This clone, designated pDH51/CPWL, was sequenced by the Maxam-Gilbert technique to confirm its suitability for expression in plants.

The plant expressible coat protein gene was then moved into a vector suitable for Agrobacterium-mediated gene transfer. Following partial digestion with EcoRI, the EcoRI to EcoRI fragment of about 1.9kb was removed from pDH51/CPWL and placed into the EcoRI site of the plasmid, pUC1813 (available from Robert Kay, Dept. of Chemistry, Washington State University, Pullman, Washington), creating the plasmid pUC1813/CPWL. A 1.9 kb fragment containing this plant expressible CMV-WL coat protein gene was removed by partial HindIII digestion and ligated into the HindIII site of the vector, pGA482 (An, 1986) (available from Gynehung An, Institute of Biological Chemistry. Washington State University). The plasmid pGA482 was previously modified to contain the plant expressible β-glucuronide gene as described in WO 89/05858, incorporated above, and the modified plasmid is referred to as pGA482/G. After cloning the expression cassette the plasmid was designated pGA482/CPWL/G (see Chart 5).

This plasmid, or its derivatives, can be transferred into Agrobacterium strains A208, C58, LBA4404, C58Z707, A4RS, A4RS(pRiB-278b) and others using methods known to those skilled in the art. Strains A208, C58, LBA4404, and A4RS are available from ATCC, 12301 Parklawn Drive, Rockville, MD. A4RS(pRiB278b) is available from Dr. F. Casse-Delbart, C.N.R.A., Routede Saint Cyr, F78000, Versailles, France. C58Z707 is available from Dr. A.G. Hepburn, University of Illinois, Urbana, IL. Agrobacterium-mediated transfer of the plant expressible CMV-WL coat protein gene is done using the procedures known to those skilled in the art or by using the methods described in a U.S. Patent application Serial No. 135,655 filed December 21, 1987 entitled "Agrobacterium Mediated Transformation of Germinating Plant Seeds", which application was refiled as PCT application PCT/US88/04464, and published on 29 June 1989 as WO 89/05859, the essential parts which are herein incorporated by reference. Transfer of this gene into plant cell can also be accomplished using other methods, such as, direct DNA uptake (Paszkowski, et al., EMBO J., 1984, 3:2717), microinjection (Crossway, et al., Mol. Gen. Genet. 202:179), electroporation (Fromm et al., Proc. Natl. Acad. Sci. U.S.A. 82:5824), or high-velocity microprojectiles (Klein, et al., Nature 327:70).

Chart 1

Nucleotide and Amino Acid Sequence of CMV-WL Coat Protein Gene

ATGGACAAATCTGGATCTCCCAATGCTAGTAGAACCTCCCGGCGTCGTCGCCCGCGTAGA
1 ---------+---------+---------+---------+---------+---------+ 60
MetAspLysSerGlySerProAsnAlaSerArgThrSerArgArgArgArgProArgArg

GGTTCTCGGTCCGCTTCTGGTGCGGATGCAGGGTTGCGTGCTTTGACTCAGCAGATGCTG
61 ---------+---------+---------+---------+---------+---------+ 120
GlySerArgSerAlaSerGlyAlaAspAlaGlyLeuArgAlaLeuThrGlnGlnMetLeu

AAACTCAATAGAACCCTCGCCATTGGTCGTCCCACTCTTAACCACCCAACCTTCGTGGGT
121 ---------+---------+---------+---------+---------+---------+ 180
LysLeuAsnArgThrLeuAlaIleGlyArgProThrLeuAsnHisProThrPheValGly

AGTGAAAGCTGTAAACCCGGTTACACTTTCACATCTATTACCCTGAAACCGCCTGAAATT
181 ---------+---------+---------+---------+---------+---------+ 240
SerGluSerCysLysProGlyTyrThrPheThrSerIleThrLeuLysProProGluIle

GAGAAAGGTTCATATTTTGGTAGAAGGTTGTCTTTGCCAGATTCAGTCACGGACTATGAT
241 ---------+---------+---------+---------+---------+---------+ 300
GluLysGlySerTyrPheGlyArgArgLeuSerLeuProAspSerValThrAspTyrAsp

AAGAAGCTTGTTTCGCGCATTCAAATCAGGGTTAATCCTTTGCCGAAATTTGATTCTACC
301 ---------+---------+---------+---------+---------+---------+ 360
LysLysLeuValSerArgIleGlnIleArgValAsnProLeuProLysPheAspSerThr

GTGTGGGTTACAGTTCGGAAAGTACCTTCATCATCCGATCTTTCCGTCGCCGCCATCTCT
361 ---------+---------+---------+---------+---------+---------+ 420
ValTrpValThrValArgLysValProSerSerSerAspLeuSerValAlaAlaIleSer

GCTATGTTTGGCGATGGTAATTCACCGGTTTTGGTTTATCAGTATGCTGCGTCCGGAGTT
421 ---------+---------+---------+---------+---------+---------+ 480
AlaMetPheGlyAspGlyAsnSerProValLeuValTyrGlnTyrAlaAlaSerGlyVal

CAGGCCAACAATAAGTTACTTTATGACCTGTCCGAGATGCGTGCTGATATCGGCGACATG
481 ---------+---------+---------+---------+---------+---------+ 540
GlnAlaAsnAsnLysLeuLeuTyrAspLeuSerGluMetArgAlaAspIleGlyAspMet

CGTAAGTACGCCGTCCTGGTTTACTCGAAAGACGATAAACTAGAGAAGGACGAGATTGCA
541 ---------+---------+---------+---------+---------+---------+ 600
ArgLysTyrAlaValLeuValTyrSerLysAspAspLysLeuGluLysAspGluIleAla

CTTCATGTCGACGTCGAGCATCAACGAATTCCTATCTCACGGATGCTCCCGACTTAG
601 ---------+---------+---------+---------+---------+------- 657
LeuHisValAspValGluHisGlnArgIleProIleSerArgMetLeuProThrEnd

Chart 2

Comparison of CMV-WL and CMV-C coat protein genes

CMV-WL ATGGACAAATCTGGATCTCCCAATGCTAGTAGAACCTCCCGGCGTCGTCG 50
CMV-C ATGGACAAATCTGAATCAACCAGTGCTGGTCGTA...ACCATCGACGTCG 47

CCCGCGTAGAGGTTCTCGGTCCG...CTTCTGGTGCGGATGCAGGGTTGC 97
TCCGCGTCGTGGTTCCCGCTCCGCCCCCTCCTCCGCGGATGCTAACTTTA 97

GTGCTTTGACTCAGCAGATGCTGAAACTCAATAGAACCCTCGCCATTGGT 147
GAGTCTTGTCGCAGCAGCTTTCGCGACTTAATAAGACGTTAGCAGCTGGT 147

CGTCCCACTCTTAACCACCCAACCTTCGTGGGTAGTGAAAGCTGTAAACC 197
CGTCCAACTATTAACCACCCAACCTTTGTAGGGAGTGAACGCTGTAGACC 197

CGGTTACACTTTCACATCTATTACCCTGAAACCGCCTGAAATTGAGAAAG 247
TGGGTACACGTTCACATCTATTACCCTAAAGCCACCAAAAATAGACCGTG 247

GTTCATATTTTGGTAGAAGGTTGTCTTTGCCAGATTCAGTCACGGACTAT 297
AGTCTTATTACGGTAAAAGGTTGTTACTACCTGATTCAGTCACGGAATAT 297

GATAAGAAGCTTGTTTCGCGCATTCAAATCAGGGTTAATCCTTTGCCGAA 347
GATAAGAAGCTTGTTTCGCGCATTCAAATTCGAGTTAATCCTTTGCCGAA 347

ATTTGATTCTACCGTGTGGGTTACAGTTCGGAAAGTACCTTCATCATCCG 397
ATTTGATTCTACCGTGTGGGTGACAGTCCGTAAAGTTCCTGCCTCCTCGG 397

ATCTTTCCGTCGCCGCCATCTCTGCTATGTTTGGCGATGGTAATTCACCG 447
ACTTATCCGTTGCCGCCATCTCTGCTATGTTCGCGGACGGAGCCTCACCG 447

GTTTTGGTTTATCAGTATGCTGCGTCCGGAGTTCAGGCCAACAATAAGTT 497
GTACTGGTTTATCAGTATGCCGCATCTGGAGTCCAAGCCAACAACAAACT 497

ACTTTATGACCTGTCCGAGATGCGTGCTGATATCGGCGACATGCGTAAGT 547
GTTGTTTGATCTTTCGGCGATGCGCGCTGATATAGGTGACATGAGAAAGT 547

ACGCCGTCCTGGTTTACTCGAAAGACGATAAACTAGAGAAGGACGAGATT 597
ACGCCGTCCTCGTGTATTCAAAAGACGATGCGCTCGAGACGGACGAGCTA 597

GCACTTCATGTCGACGTCGAGCATCAACGAATTCCTATCTCACGGATGCT 647
GTACTTCATGTTGACATCGAGCACCAACGCATTCCCACATCTGGAGTGCT 647

CCCGACTTAG 657
CCCAGTCTGA 657

Chart 3

Comparison of CMV-WL and CMV-C coat proteins

CMV-WL MDKSGSPNASRTSRRRRPRRGSRSA.SGADAGLRALTQQMLKLNRTLAIG 49
|||| |  | |   ||||||||||| | |||  | | ||   || ||| |
CMV-C MDKSESTSAGR.NHRRRPRRGSRSAPSSADANFRVLSQQLSRLNKTLAAG 49

RPTLNHPTFVGSESCKPGYTFTSITLKPPEIEKGSYFGRRLSLPDSVTDY 99
||| ||||||||| | ||||||||||||| |   || | || |||||| |
RPTINHPTFVGSERCRPGYTFTSITLKPPKIDRESYYGKRLLLPDSVTEY 99

DKKLVSRIQIRVNPLPKFDSTVWVTVRKVPSSSDLSVAAISAMFGDGNSP 149
|||||||||||||||||||||||||||||| ||||||||||||| || ||
DKKLVSRIQIRVNPLPKFDSTVWVTVRKVPASSDLSVAAISAMFADGASP 149

VLVYQYAASGVQANNKLLYDLSEMRADIGDMRKYAVLVYSKDDKLEKDEI 199
|||||||||||||||||| ||| |||||||||||||||||||| || ||
VLVYQYAASGVQANNKLLFDLSAMRADIGDMRKYAVLVYSKDDALETDEL 199

ALHVDVEHQRIPISRMLPT* 219
 |||| |||||| |  ||
VLHVDIEHQRIPTSGVLPV* 219

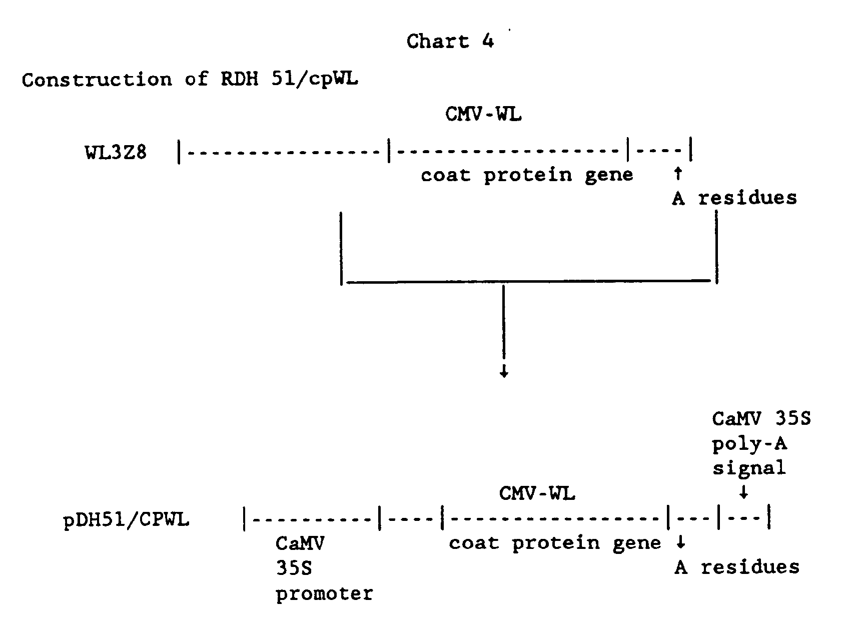
Chart 4

Construction of RDH 51/cpWL

CMV-WL

WL3Z8 |----------------|------------------|---|

coat protein gene ↑

A residues


CaMV 35S

poly-A

signal

CMV-WL ↓

pDH51/CPWL |----------|----|-----------------|---|---|

CaMV coat protein gene ↓

35S A residues

promoter

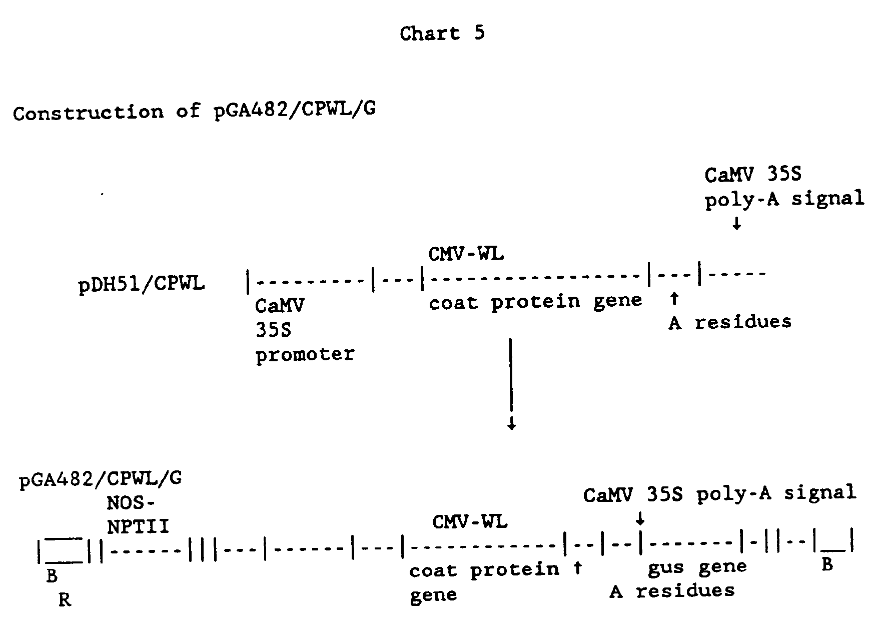
Chart 5

Construction of pGA482/CPWL/G

CaMV 35S
poly-A signal
↓
CMV-WL
pDH51/CPWL
CaMV coat protein gene ↑
35S A residues
promoter

pGA482/CPWL/G
NOS- CaMV 35S poly-A signal
NPTII CMV-WL ↓
B coat protein ↑ gus gene B
R gene A residues

## Claims

1. The coat protein gene from the WL strain of cucumber mosaic virus, having the sequence shown in Chart 1.

2. A plant transformation vector comprising the coat protein gene defined in claim 1, the 35S promoter of cauliflower mosaic virus, and the polyadenylation signal of the cauliflower mosaic 35S gene.

3. A bacterial cell containing the plant transformation vector of claim 2.

4. A transformed plant cell containing the plant transformation vector of claim 2.

5. A cucurbitaceae plant comprising transformed cells of claim 4.

6. A solanaceae plant comprising transformed cells of claim 4.

7. A process for producing a virus-resistant cucubitaceae plant, comprising propagating a cucurbitaceae plant expressing the coat protein gene defined in claim 1.

8. A process for producing a virus-resistant solanaceae plant, comprising propagating a solanaceae plant expressing the coat protein gene defined in claim 1.

## Patentansprüche

1. Hülleproteingen aus dem WL-Stamm von Gurkenmosaikvirus mit der in Schema 1 dargestellten Sequenz.

2. Pflanzentransformationsvektor mit dem Hülleproteingen gemäß Anspruch 1, dem 35S-Promotor von Blumenkohlmosaikvirus und dem Polyadenylierungssignal des Blumenkohlmosaik 35S-Gens.

3. Bakterienzelle mit dem Pflanzentransformationsvektor gemäß Anspruch 2.

4. Transformierte Pflanzenzelle mit dem Pflanzentransformationsvektor nach Anspruch 2.

5. Cucurbitaceae-Pflanze mit transformierten Zellen nach Anspruch 4.

6. Solanaceae-Pflanze mit transformierten Zellen nach Anspruch 4.

7. Verfahren zur Produktion einer virusresistenten Cucurbitaceae-Pflanze durch Fortpflanzung einer das Hülleproteingen nach Anspruch 1 exprimierenden Cucurbitaceae-Pflanze.

8. Verfahren zur Produktion einer virusresistenten Solanaceae-Pflanze durch Fortpflanzung einer das Hülleproteingen nach Anspruch 1 exprimierenden Solanaceae-Pflanze.

## Revendications

1. Gène de protéine d'enveloppe provenant de la souche WL du virus de la mosaïque du concombre, ayant la séquence représentée sur le schéma 1.

2. Vecteur de transformation de végétaux comprenant le gène de protéine d'enveloppe défini suivant la revendication 1, le promoteur 35S du virus de la mosaïque du chou-fleur, et le signal de polyadénylation du gène 35S du virus de la mosaïque du chou-fleur.

3. Cellule bactérienne contenant le vecteur de transformation de végétaux suivant la revendication 2.

4. Cellule végétale transformée contenant le vecteur de transformation de végétaux suivant la revendication 2.

5. Plante de la famille des Cucurbitaceae, comprenant des cellules transformées suivant la revendication 4.

6. Plante de la famille des Solanaceae, comprenant des cellules transformées suivant la revendication 4.

7. Procédé de production d'une plante de la famille des Cucurbitaceae, résistante aux virus, comprenant la culture d'une plante de la famille des Cucurbitaceae effectuant l'expression du gene de protéine d'enveloppe défini suivant la revendication 1.

8. Procédé de production d'une plante de la famille des Solanaceae, résistante aux virus, comprenant la culture d'une plante de la famille des Solanaceae effectuant l'expression du gène de protéine d'enveloppe défini suivant la revendication 1.
